# EUROPEAN PATENT SPECIFICATION

(11) **EP 3 652 246 B1**
(45) Date of publication and mention of the grant of the patent: **10.05.2023**
(21) Application number: 17917231.7
(22) Date of filing: 13.07.2017
(51) Int. Cl.: C08L 23/12, A61J 1/00

(54) **STERILIZABLE MEDICAL PACKAGING WITH LIVING PORES**
STERILISIERBARE MEDIZINISCHE VERPACKUNG MIT LEBENDEN POREN
EMBALLAGE MÉDICAL STÉRILISABLE À PORES VIVANTS

(43) Date of publication of application: 20.05.2020
(73) Proprietor: Enzpire Industry Co., Ltd., Bangkok 10150 (TH)
(72) Inventor: WICHITAMORNLOET, Arthorn, Bangkhuntian Bangkok 10150 (TH)
(74) Representative: Cabinet Chaillot
(86) International application number: PCT/TH2017/000054
(87) International publication number: WO 2019/013715

(56) References cited:
- EP-A1- 2 444 453
- EP-B1- 0 973 824
- EP-B1- 3 030 607
- WO-A1-2013/054929
- WO-A1-2015/083665
- CN-A- 102 286 169
- CN-A- 106 700 224
- CN-A- 106 795 346
- JP-A- 2015 163 687
- US-A1- 2003 207 137

## Description

### Field of the invention

The present invention relates to the field of polymer science and technology with special emphasis on the production of sterilized plastic packaging for products that need to be sterilized, especially medical products.

### Background of the invention

A medical packaging is a sterile packaging, commonly made of plastic, with good barrier to microbes or other contaminates to maintain the sterility of medical devices inside after the sterilization process. Commonly, the medical packaging in the market are classified into 2 categories according to their pore structures as follows: (1) A Non-Porous Medical Packaging: which is commonly made from commoditized thermoplastic polymers such as but not limited to, polyethylene, polypropylene in variety of grades as homopolymers, copolymers, heterophasic polymers, and blends thereof, processed by an extruder such as, but not limited to, blown film extruder, T-die cast film extruder, or calendaring extrusion to form either single-layered, or multi-layered film-liked substrate that then further fabricated to be a medical packaging. Without any opened dead pore, non-fibrous web contained structure, these types of medical packaging give an excellent barrier property in protecting medical devices inside. Unfortunately, their dense structures may worsen their ability to pass some methods of sterilizations, especially sterilization methods by ethylene oxide gas or steam sterilization, whereby the ability of sterilant gases to permeate through the packaging is the key factor, so a radiation sterilization is a common alternative for this kind of medical packaging; and (2) A Porous Medical Packaging: which normally consist of randomly laid webs that contain opened dead pores. The pores allow sterilant gases, such as ethylene oxide or steam, to permeate through its structure. This type of medical packaging can undergo more sterilization methods than the one mentioned above. However, the web has a broad pore size distribution and variety of shapes which may cause more risks of losing their sterility as some microbes, impurities, or contaminants can penetrate through the packaging via its over-tolerance, big pores. Thus, a rigorous hygienic storage is needed to ensure and maintain its sterility and to prolong the products shelf life. The medical packaging with fibrous webs can be further classified into 2 categories according to the types of fibrous materials as follows: (2a) Medical packaging with a paper web made of natural fibers of wood pulps or plant's fiber like cotton fiber, whose chemical structures mainly are cellulose. Cellulose is sensitive to water so it likes to absorb water that will diminish its physical strength, and is spoiled by microbes that consequently lead to losing their barrier functionality and sterility; and (2b) Medical packaging with a thermoplastic fiber web, especially a high density polyethylene web, which can eliminate the disadvantage of a natural short fiber kraft paper described above. Nevertheless, some drawback about bonding and opening the packaging is concerned.

Below are some examples of common sterilization methods:
- Radiation Sterilization: Using Gamma rays or the electromagnetic energy emitting from radioisotrope cobalt-60 to penetrate packaging and sterilize medical devices inside, such as syringe, needle, IV sets;
- Heat Sterilization: High temperature, high pressure water vapor in a steam autoclave is the most common method to sterilize medical instruments or devices that can withstand high heat conditions such as forceps or other stainless steel devices by exposure to 121 - 134 degree Celsius (°C) steam in a pressurized chamber; and
- Chemical Sterilization: Ethylene oxide gas is most widely used as chemical sterilant permeating through a porous packaging to sterilize medical devices inside. This process is suitable for heat or moisture sensitive articles such as electronic or low melting point plastic and rubber parts or devices.

Clearly, the first packaging with densed structure has an excellent barrier property to keep its sterility, but cannot undergo all methods of sterilization, especially by sterilants that adequate porosity is an essential factor, so a gramma radiation is an alternative method which has some drawbacks like much more rigorious safety controls in sterilization plant, radio-active accident concerns, that limits the use of this type of packaging, while other plastic sterile medical packaging, comprising fibrous webs, either a natural celluosic kraft paper or a synthetic thermoplastic webs, enable sterilization by steam or ethylene oxide gas, which are much more versatile to process safety even in a general hospital. Nevertheless, its versatility to sterile with all common sterilizations methods is sacrifice with its worse barrier function. As its board distribution of pore size, which the over large-sized of pores will increae a risk of microbial penetration and then lose its sterility. Thus, a sterilization method selected must be compatible to the packaging structure used and vice versa.

Thus, this novel sterilizable medical packaging with living pores, particularly with living pores, is invented to give a smarter packaging with excellent barrrier and reliable sterility, which can also undergo all types of sterilization methods.

### Detailed description of the invention

The present invention is related to a sterilizable medical packaging with living pores comprising a set of living pores, wherein the packaging is preferably a plastic film packaging, but without any open dead pores or containing non-fibrous web made of semi-crystalline polyolefin materials. Presently, the most common thermoplastic materials used in plastic packaging are polyethylene, polypropylene, polybutylene, and especially isotactic polypropylene which is used as main raw material. Nevertheless, these commodity plastics which undergo common processing by any commercial film/sheet forming processes-like blown film extrusion, cast film extrusion, with or without further orientation stretching-will not provide a sterile medical packaging that can undergo and withstand all common sterilization methods especially by steam sterilization and ethylene oxide sterilization methods as their dense structures would often obstruct steams or sterilant gas permeability, so the pressure will damage or deform the packaging during in sterilization procedure.

To overcome this problem, the sterilizable medical packaging with living pores according to this invention, therefore, comprises a unique formulation of polyolefin blend with specific additives are proposed. A blend of polyolefins such as, but not limited to, polyethylene, polypropylene, polybutylene, copolymer of C2-C8 alpha olefins, preferably comprising at least 50 percent by weight of isotactic polypropylene homopolymer, most preferably high molecular weight, high crystallinity isotactic polypropylene homopolymer with low melt flow index of less than 5; a functional additive comprising 100-2,500 ppm of nucleating agent such as, but not limited to, sorbitol derivatives, 1,3,5-benzenetrisamide derivatives, which can induce very fine crystalline lattices of isotactic polypropylene that toughens the packaging structure by increasing tensile strength of the film substrate, especially in transverse direction, while their percentage of elongation remains high; and 0.1-2 percent by weight of hydrophilic filler selected from silica with average size of 5 microns or less, polyethylene oxide with molecular weight of more than 1,000 or blends thereof to provide amphiphilic paths improving gas permeability, especially water-vapor transmission rate (e.g. in one embodiment, the water vapor transmission rate of said packaging at modified negative pressure, 45 kPa, is at least 10g/m²/day), and other common additives as required.

In one preferred embodiment, the above compound mixtures are heated and plasticized with 180 -250 °C in an extruder, such as, but not limited to, a blown film extruder to form a film-like substrate material with a cooling rate in range of 1-10°C/second, and undergo common process, similar to other packaging, such as treating with corona discharge, printing, slit sealing and cutting, to become a sterilizable medical packaging with living pores according to this invention.

With the specific formula and film forming process's condition, the resulting sterilizable medical packaging with living pores according to this invention has a unique structure that gives an excellent barrier to microbes or other impurities as its structure has excluded the open dead pores. The ratio of tensile strength of said packaging in transverse direct per machine direction is greater than 0.7. the ratio of an elongation of said packaging in machine direction per transverse direction is greater than 1, and the elongation in machine direction is greater than 200%.

With its heterophase structure, in one embodiment, comprising a rigid tough network of crystalline lattice of isotactic polypropylene, which attributes the overall strength of the plastic packaging, meanwhile the other soft, elastic amorphous region that disperses all around the interface of the crystalline network plays a main role in gas permeability. In addition, with the balance of both regions, a rigid, tough crystalline network and a soft elastic amorphous interface gives this unique heterophase structure an extraordinary physical property and performance similar to having a set of living pores. The resulting sterilizable medical packaging with living pores will provide a good barrier where it can be stored in common atmosphere or open air, but under controlled pressure in sterilized chamber, especially in negative pressure during evacuation process, the interstitial volume between crystalline interface will enlarge, enabling the sub-micrometer pores that are dispersing thoroughly through the matrix to allow water vapor or sterilant gas to permeate through the packaging to sterilize any products that need to be sterilized, such as medical products. Without dead, open-pores, this sterilizable medical packaging with living pores according to this invention will prolong the product shelf life and give a better performance in maintaining sterility even in severe conditions like in war services or field applications.

Although this invention has been disclosed in the context of certain embodiment and examples, it will be understood by those skilled in the art that the present invention extends beyond the specifically disclosed embodiment to other alternative embodiments and/or uses of the invention and obvious modifications and equivalents thereof.

## Claims

1. - A sterilizable medical packaging with living pores comprising:
- a polyolefin blend comprising at least 50 percent by weight of an isotactic polypropylene homopolymer;
- 100-2,500 ppm of a nucleating agent; and
- 0.1-2 percent by weight of a hydrophilic filler;
wherein the above compositions are mixed and plasticized at temperature in range of 180-250 °C and cooled down with cooling rate in range of 1-10 °C per second to form a sterilizable medical substrate film packaging according to this invention.

2. - The sterilizable medical packaging with living pores according to claim 1, wherein the polyolefins of the polyolefin blend are seleted among polyethylene, polypropylene, polybutylene and copolymer of C2-C8 alpha olefins.

3. - The sterilizable medical packaging with living pores according to claim 1 wherein the isotactic polypropylene homopolymer is a high molecular weight, high crystallinity isotactic polypropylene homopolymer with low melt flow index of less than 5.

4. - The sterilizable medical packaging with living pores according to claim 1 wherein the nucleating agent is selected from a group of sorbitol derivatives, or 1,3,5- benzenetrisamide derivatives, or their combinations thereof.

5. - The sterilizable medical packaging with living pores according to claim 1 wherein the hydrophilic filler is selected from a silica, or polyethylene oxide or their combinations thereof.

6. - The sterilizable medical packaging with living pores according to claim 5, wherein the silica has an average size of 5 microns or less.

7. - The sterilizable medical packaging with living pores according to claim 5, wherein the polyethylene oxide has a molecular weight of more than 1,000.

8. - The sterilizable medical packaging with living pores according to claim 1 wherein the packaging comprises non-fibrous, non-opened dead pores plastic film packaging.

9. - The sterilizable medical packaging with living pores according to claim 1 wherein the ratio of tensile strength of said packaging's film substrate in transverse direct per machine direction is greater than 0.7.

10. - The sterilizable medical packaging with living pores according to either of claim 1 or 9 wherein the ratio of an elongation of said packaging's film substrate in machine direction per transverse direction is greater than 1, and the elongation in machine direction is greater than 200%.

11. - The sterilizable medical packaging with living pores according to any of claims 1-10 wherein the average pore size is less than 1 micron.

12. - The sterilizable medical packaging with living pores according to any of claims 1-11 wherein the water vapor transmission rate of said packaging at modified negative pressure, -45 kPa, is at least 10g/m²/day.

## Patentansprüche

1. - Sterilisierbare medizinische Verpackung mit Lebendporen, die umfasst:
- eine Polyolefinmischung, die zumindest 50 Gewichtsprozent isotaktisches Polypropylenhomopolymer umfasst;
- 100 bis 2500 ppm Keimbildner; und
- 0,1 bis 2 Gewichtsprozent hydrophilen Füllstoff;
wobei die obigen Zusammensetzungen vermischt und bei einer Temperatur im Bereich von 180 bis 250 °C plastifiziert werden und mit einer Kühlrate im Bereich von 1 bis 10 °C pro Sekunde abgekühlt werden, um eine sterilisierbare medizinische Substratfolienverpackung gemäß dieser Erfindung auszubilden.

2. - Sterilisierbare medizinische Verpackung mit Lebendporen nach Anspruch 1, wobei die Polyolefine der Polyolefinmischung aus Polyethylen, Polypropylen, Polybutylen und Copolymer von C2-C8-alpha-Olefinen ausgewählt sind.

3. - Sterilisierbare medizinische Verpackung mit Lebendporen nach Anspruch 1, wobei das isotaktische Polypropylenhomopolymer ein hochmolekulares, hochkristallines isotaktisches Polypropylenhomopolymer mit einem geringen Schmelzflussindex von weniger als 5 ist.

4. - Sterilisierbare medizinische Verpackung mit Lebendporen nach Anspruch 1, wobei der Keimbildner aus einer Gruppe von Sorbitderivaten oder 1,3,5-Benzoltrisamidderivaten oder Kombinationen dieser ausgewählt ist.

5. - Sterilisierbare medizinische Verpackung mit Lebendporen nach Anspruch 1, wobei der hydrophile Füllstoff aus einem Siliciumdioxid oder Polyethylenoxid oder Kombinationen davon ausgewählt ist.

6. - Sterilisierbare medizinische Verpackung mit Lebendporen nach Anspruch 5, wobei das Siliciumdioxid eine durchschnittliche Größe von 5 Mikrometern oder weniger aufweist.

7. - Sterilisierbare medizinische Verpackung mit Lebendporen nach Anspruch 5, wobei das Polyethylenoxid ein Molekulargewicht von mehr als 1000 aufweist.

8. - Sterilisierbare medizinische Verpackung mit Lebendporen nach Anspruch 1, wobei die Verpackung eine nichtfasrige Kunststofffolienverpackung mit nicht offenen toten Poren umfasst.

9. - Sterilisierbare medizinische Verpackung mit Lebendporen nach Anspruch 1, wobei das Verhältnis von Zugfestigkeit des Foliensubstrats der Verpackung in Querrichtung in Bezug auf die Maschinenrichtung mehr als 0,7 beträgt.

10. - Sterilisierbare medizinische Verpackung mit Lebendporen nach Anspruch 1 oder 9, wobei das Verhältnis einer Dehnung des Foliensubstrats der Verpackung in Maschinenrichtung in Bezug auf die Querrichtung mehr als 1 beträgt und die Dehnung in Maschinenrichtung mehr als 200 % beträgt.

11. - Sterilisierbare medizinische Verpackung mit Lebendporen nach einem der Ansprüche 1 bis 10, wobei die durchschnittliche Porengröße weniger als 1 Mikrometer beträgt.

12. - Sterilisierbare medizinische Verpackung mit Lebendporen nach einem der Ansprüche 1 bis 11, wobei die Wasserdampfdurchlässigkeitsrate der Verpackung bei modifiziertem Unterdruck, -45 kPa, zumindest 10 g/m²/Tag beträgt.

## Revendications

1. - Emballage médical stérilisable ayant des pores vivants, comprenant :
- un mélange de polyoléfines comprenant au moins 50 pour cent en poids d'un homopolymère de polypropylène isotactique ;
- 100 - 2500 ppm d'un agent de nucléation ; et
- 0,1 - 2 pour cent en poids d'une charge hydrophile ;
les compositions ci-dessus étant mélangées et plastifiées à une température dans la plage de 180 - 250°C et refroidies à une allure de refroidissement dans la plage de 1 - 10 °C par seconde pour former un emballage à film de substrat médical stérilisable selon cette invention.

2. - Emballage médical stérilisable ayant des pores vivants, selon la revendication 1, dans lequel les polyoléfines du mélange de polyoléfines sont choisies parmi le polyéthylène, le polypropylène, le polybutylène et un copolymère d'alpha-oléfines en C2-C8.

3. - Emballage médical stérilisable ayant des pores vivants, selon la revendication 1, dans lequel l'homopolymère de polypropylène isotactique est un homopolymère de polypropylène isotactique de masse moléculaire élevée et à cristallinité élevée avec un faible indice de fusion de moins de 5.

4. - Emballage médical stérilisable ayant des pores vivants, selon la revendication 1, dans lequel l'agent de nucléation est choisi dans un groupe de dérivés de sorbitol, ou de dérivés de 1,3,5-benzènetrisamide, ou leurs combinaisons.

5. - Emballage médical stérilisable ayant des pores vivants, selon la revendication 1, dans lequel la charge hydrophile est choisie dans une silice, ou un poly(oxydee d'éthylène), ou leurs combinaisons.

6. - Emballage médical stérilisable ayant des pores vivants, selon la revendication 5, dans lequel la silice a une taille moyenne de 5 microns ou moins.

7. - Emballage médical stérilisable ayant des pores vivants, selon la revendication 5, dans lequel le poly(oxyde d'éthylène) a une masse moléculaire de plus de 1000.

8. - Emballage médical stérilisable ayant des pores vivants, selon la revendication 1, dans lequel l'emballage comprend un emballage de film plastique à pores morts non ouverts, non fibreux.

9. - Emballage médical stérilisable ayant des pores vivants, selon la revendication 1, dans lequel le rapport de la résistance à la traction dudit substrat de film de l'emballage dans la direction transversale à cette même résistance dans le sens machine est supérieur à 0,7.

10. - Emballage médical stérilisable ayant des pores vivants, selon l'une des revendications 1 ou 9, dans lequel le rapport d'un allongement dudit substrat de film de l'emballage dans le sens machine à l'allongement dans la direction transversale est supérieur à 1, et l'allongement dans le sens machine est supérieur à 200 %.

11. - Emballage médical stérilisable ayant des pores vivants, selon l'une quelconque des revendications 1 à 10, dans lequel la taille moyenne de pores est inférieure à 1 micron.

12. - Emballage médical stérilisable ayant des pores vivants, selon l'une quelconque des revendications 1 à 11, dans lequel le taux de transmission de vapeur d'eau dudit emballage à une pression négative modifiée, -45 kPa, est d'au moins 10 g/m²/jour.
